# EUROPEAN PATENT APPLICATION

(11) **EP 2 083 010 A1**
(43) Date of publication of application: **29.07.2009**
(21) Application number: 08150100.9
(22) Date of filing: 08.01.2008
(51) Int. Cl.: C07D 471/04, A61K 31/437, A61P 31/04

(54) **Polymorphic Forms of Moxifloxacin hydrochloride and processes for preparation thereof**

(71) Applicant: Chemo Ibérica, S.A., 08028 Barcelona (ES)
(72) Inventor: Ventimiglia, Gianpiero, 72021, Villafranca Fontana (BR) (IT); Magrone, Domenico, 20128, Milano (IT); Castaldi, Graziano, 28072, Briona (NO) (IT)
(74) Representative: Barlocci, Anna

(57) **Abstract**

Polymorphic hydrate crystalline forms of Moxifloxacin hydrochloride were found, referred to hereinafter as polymorphic Form α1 and Form α2. Furthermore, the present invention is directed to processes for the preparation of these crystalline forms, to pharmaceutical compositions comprising them, as well as their use.

## Description

### Field of the invention

The present invention relates to novel crystalline modifications of Moxifloxacin hydrochloride, to processes for preparing them, to pharmaceutical compositions containing them, as well as their use.

### Background of the invention

Moxifloxacin, a fluoroquinolone compound, is a synthetic broad spectrum antibacterial agent, widely used in the treatment of infections by antibiotic-resistant bacteria. Moxifloxacin is one of the most active quinolones against bacteria which are resistant to penicillins and macrolides.

Moxifloxacin hydrochloride shows a low toxicity and it has been shown to be active against most strains of microorganisms such as aerobic Gram-positive microorganism, including *Staphylococcus aureus, Streptococcus pneumoniae* (penicillin-susceptible strains) and *Streptococcus pyogenes;* aerobic Gram-negative microorganism, including *Haemophilus influenzae, Haemophilus parainfluenzae, Klebisiella pneumoniae* and *Moraxella catarrhalis;* and against the respiratory disease causing pathogens like *Chlamydia pneumoniae, Mycoplasma pneumoniae* and *Mycobacterium tuberculosis.*

Moxifloxacin hydrochloride is a compound of Formula (I), 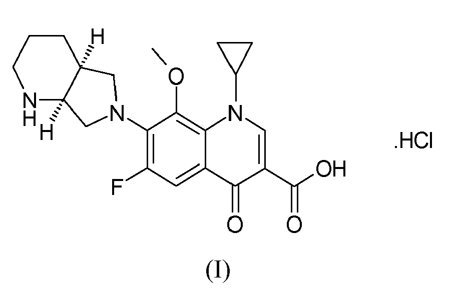 chemically known as 1-cyclopropyl-6-fluoro-1,4-dihydro-8-methoxy-7-[(4aS,7aS)-octahydro-6H-pyrrolo[3,4-b]pyridin-6-yl]-4-oxoquinoline-3-carboxylic acid hydrochloride.

Moxifloxacin hydrochloride, related compounds and processes to obtain them are disclosed in US 4,990,517, US 5,639,886 and EP-A-550903.

Polymorphism is the property of molecules and molecular complexes to assume more than one crystalline or amorphous form in the solid state. The different structures are referred to as polymorphs, polymorphic modification or polymorphic forms. Substances are known which only appear in a single crystal form; in addition, however, there are also substances which can form two, three or even more polymorphic crystal modifications. In general, polymorphism is caused by the ability of the molecule of a substance to change its conformation or to form different inter molecular and intramolecular interactions, particularly hydrogen bonds, which is reflected in different atom arrangements in the crystal lattices of different polymorphs. Accordingly, polymorphs are distinct solids sharing the same molecular formula, having distinct advantageous and/or disadvantageous physical properties compared to other forms in the polymorphic family.

The morphology and polymorphology of organo-chemical active substances is of great importance to the chemical and pharmaceutical development thereof. One crystalline form may provide significant advantages over other crystalline forms. Furthermore, a particular process suitable for one crystalline form may also provide drug manufacturers several advantages such as economically or environmentally suitable solvents or process, or higher purity or yield of the desired product.

The relevant polymorphism of an organo-chemical substance is always unpredictable in respect of the number of crystal modifications, the stability thereof and their behaviour in a living organism.

The different polymorphs of a substance possess different energies of the crystal lattice and, thus, they show different physical properties of the solid state such as form, density, melting point, colour, stability, dissolution rate, milling facility, granulation, compacting etc. These differences in morphology and polymorphism may have drastic effects on the flowability of the milled solid (flowability affects the ease with which the material is handled during processing into a pharmaceutical product), development, transport stability and storage stability of individual administration forms, on the ability to produce different administration forms, on their application, on the solubility in polar or non-polar, protic or aprotic solvents, on solubility in aqueous solution, on solubility in the gastric juices, on solubility in blood serum, and finally on bio-availability. The rate of dissolution of an active ingredient in a patient's stomach fluid can have therapeutic consequences since it imposes an upper limit on the rate at which an orally-administered active ingredient can reach the patient's bloodstream. The rate of dissolution is also a consideration in formulating syrups, elixirs and other liquid medicaments. Other important properties of polymorphic forms relate to the ease of processing the form into pharmaceutical dosages, as the tendency of a powdered or granulated form to flow and the surface properties that determine whether crystals of the form will adhere to each other when compacted into a tablet. The polymorphic form may give rise to thermal behavior different from that of the amorphous material or another polymorphic form. Thermal behavior is measured in the laboratory by such techniques as capillary melting point, Differential Scanning Calorimetry (DSC) and can be used to distinguish some polymorphic forms from others. A particular polymorphic form may also give rise to distinct spectroscopic properties that may be detectable by X-Ray Powder Diffraction (XRPD).

The same also applies in respect of the physical and chemical properties of Moxifloxacin hydrochloride.

According to US 5,849,752, two crystalline modifications of anhydrous Moxifloxacin hydrochloride and monohydrate Moxifloxacin hydrochloride, herein referred to for convenience as Form I and Form II, and the processes for preparing them are disclosed.

In WO 2004091619, a polymorphic form of anhydrous Moxifloxacin hydrochloride, designated as Form III, has been described.

An amorphous form of Moxifloxacin hydrochloride is disclosed in WO 2004039804, along with a process to prepare the same by using a spray-drying or freeze-drying technique of a Moxifloxacin hydrochloride solution.

In WO 2005054240, two anhydrous crystalline forms of Moxifloxacin hydrochloride, designated as Form A and Form B, and the preparation thereof are described.

WO 2006134491 provides a crystalline form of Moxifloxacin hydrochloride, designated as Form A, having a moisture content of 1 to 2 % w/w amounting closely to hemihydrate. A process for the preparation of this crystalline Form A, are also provided, involving an environmentally hazard boron difluoride chelate.

WO 2007010555 provides two anhydrous crystalline forms of Moxifloxacin hydrochloride, designated as Form X and Form Y, and processes for their preparation.

The discovery of novel polymorphic forms of a pharmaceutically useful compound provides a novel opportunity to improve the performance characteristics of a pharmaceutical product. It enlarges the repertoire of materials that a formulation scientist has available for designing a pharmaceutical dosage form, or a drug with a targeted release profile, or other desired characteristics, such as flowability and suitable rate of dissolution in aqueous fluid. Therefore, the preparation of novel and stable crystalline forms of Moxifloxacin hydrochloride is desirable.

An object of the present invention is to provide novel, stable and easily formulated polymorphic crystalline forms of Moxifloxacin hydrochloride, processes for preparing them, pharmaceutical compositions containing them, as well as their use.

### Summary of the invention

It has now been found that Moxifloxacin hydrochloride can be converted into new hydrate crystalline modifications, which are distinguished by an increased stability, compared with the known forms, and are particularly suitable for the preparation of stable pharmaceutical formulations.

In one aspect, the invention provides a novel hydrate crystalline form of Moxifloxacin hydrochloride, herein referred to as Form α1. The hydrate crystalline Form α1 of Moxifloxacin hydrochloride is characterized by an XRPD pattern comprising peaks at about 5.6, 7.0, 8.3, 9.9, 14.3, 15.4, 17.2, 20.2, 23.4, 26.4, 27.3, 29.0 ± 0.2° 2θ; and a DSC endotherm maximum peak at about 250 °C.

In another aspect, the invention provides a process for preparing the hydrate crystalline Form α1 of Moxifloxacin hydrochloride comprising the steps of:
a) suspending Moxifloxacin hydrochloride in aqueous hydrochloric acid;
b) heating until dissolution;
c) cooling to obtain a precipitate;
d) recovering the product.

In another aspect, the invention provides a novel hydrate crystalline form of Moxifloxacin hydrochloride, herein referred to as Form α2. The hydrate crystalline Form α2 of Moxifloxacin hydrochloride is characterized by an XRPD pattern comprising peaks at about 5.7, 7.1, 8.4, 10.2, 14.4, 16.9, 19.1, 21.5, 26.3, 27.2 ± 0.2° 2θ; and a DSC endotherm maximum peak at about 253 °C.

In another aspect, the invention provides a process for preparing the hydrate crystalline Form α2 of Moxifloxacin hydrochloride comprising the steps of:
a) suspending Moxifloxacin hydrochloride in a mixture of water and at least a suitable aprotic polar solvent;
b) heating until dissolution;
c) cooling to obtain a precipitate;
d) recovering the product.

The hydrate crystalline Form α1 and α2 of Moxifloxacin hydrochloride are useful in the treatment of infections.

In another aspect, the invention provides a hydrate crystalline Form α1 of Moxifloxacin hydrochloride for use as a medicament.

In another aspect, the invention provides the use of a hydrate crystalline Form α1 of Moxifloxacin hydrochloride for the preparation of a medicament for the treatment of infections.

In another aspect, the invention provides a hydrate crystalline Form α2 of Moxifloxacin hydrochloride for use as a medicament.

In another aspect, the invention provides the use of a hydrate crystalline Form α2 of Moxifloxacin hydrochloride for the preparation of a medicament for the treatment of infections.

In another aspect, the invention provides a pharmaceutical composition comprising the hydrate crystalline Forms α1 or α2 of Moxifloxacin hydrochloride, optionally together with at least one pharmaceutically acceptable excipient.

### Brief Description of the Drawings

Figure 1 provides an XRPD pattern of Form α1 of Moxifloxacin hydrochloride;
Figure 2 provides a DSC thermogram of Form α1 of Moxifloxacin hydrochloride; It shows an endothermic maximum peak at 250.43°C, with an integral of 171.25 mJ normalized 74.26 Jg⁻¹.
Figure 3 provides an XRPD pattern of Form α2 of Moxifloxacin hydrochloride;
Figure 4 provides a DSC thermogram of Form α2 of Moxifloxacin hydrochloride. It shows an endothermic maximum peak at 253.05°C, with an integral of 314.20 mJ normalized 131.36 Jg⁻¹.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The term "hydrate" refers to a molecular complex of Moxifloxacin hydrochloride with one or more water molecules, wherein the water retains its molecular state as water and is either absorbed, adsorbed or contained within a crystal lattice of the substrate molecule of Moxifloxacin hydrochloride.

The term "about" encompasses the range of experimental error that may typically occurs in a measurement.

The term "excipient" herein means any substance, not itself a therapeutic agent, used as a carrier or vehicle for delivery of a therapeutic agent to a subject or added to a pharmaceutical composition to improve its handling or storage properties or to permit or facilitate formation of a dose unit of the composition into a discrete article such as a tablet, capsule, pill, powder, granule, pellet, lozenge, pastille, elixir, syrup, solution, suspension, emulsion, drop, lotion, spray, tincture, cream, ointment, gel, unguent, suppository and transdermal devices for oral, enteral, parenteral or topical administrations.

The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient.

The present invention provides Moxifloxacin hydrochloride in novel hydrate crystalline forms, herein designated as Form α1 and Form α2, and processes for preparing them.

The novel Forms α1 and α2 of Moxifloxacin hydrochloride are different from the forms well known. These crystalline forms are non hygroscopic crystalline forms. They do not absorb moisture under adverse storage conditions and during pharmaceutical processing of the active ingredient into medicament forms. They showed good handling properties and greater stability. This has considerable advantages in the formulation of a medicament. By using a non hygroscopic crystalline form, a satisfactory dosing accuracy is achieved during the preparation of medicaments, which increases safety and therefore minimizes the risk to the patient. Further, Forms α1 and α2 of Moxifloxacin hydrochloride are characterized by an ease and industrially applicable method of preparation to obtain a high quality product.

These novel crystalline Forms α1 and α2 have a water content of about 3.5-4.2 % w/w as analysed by Karl Fisher method (determined in methanol).

XRPD analysis and DSC thermal analysis were used to characterize polymorphic forms of Moxifloxacin hydrochloride.

XRPD analysis was performed on a APD 2000 Ital Structures diffractometer at room temperature, using a CuKα tube (40 kV, 30 mA, λ = 1.5418 Å) as the X-ray source. Data collection was made in 2θ step scan mode, at a scan speed of 0.02°/s in the range of 3° to 40° in 2θ. Approximately 100 mg samples were accurately grinded and placed on an aluminium sampler.

DSC thermal analysis was performed on a Mettler Toledo Star 822 differential scanning calorimeter. Approximately 2-5 mg samples were placed in aluminium pans and heated from 30 to 250°C in a dry nitrogen atmosphere at a heating rate of 10°C/minute.

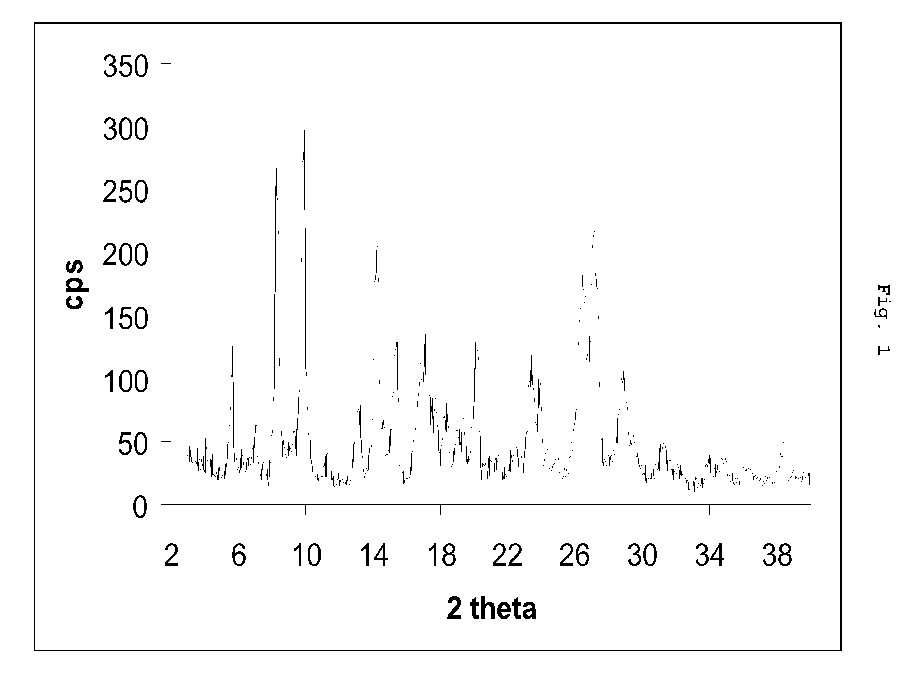

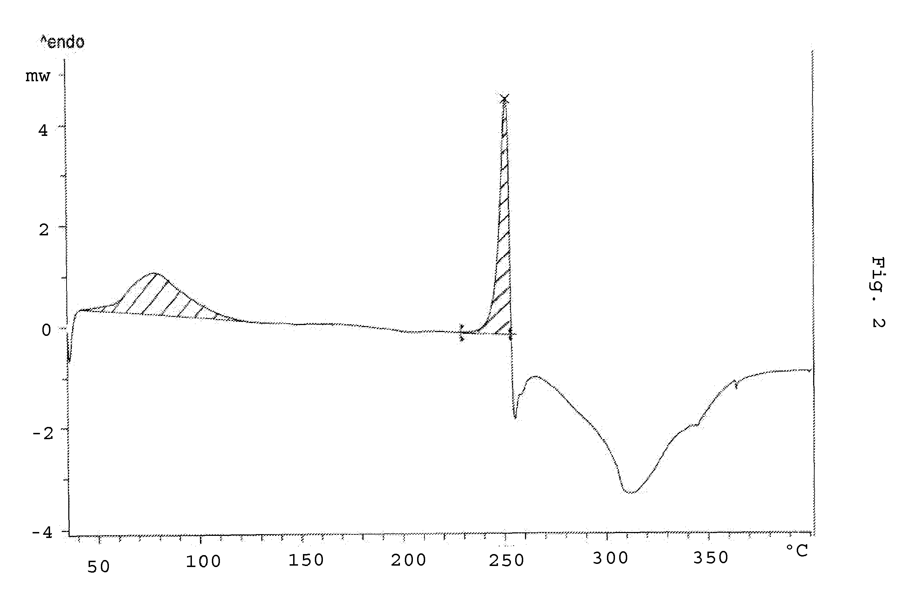

In one aspect, the invention provides a novel hydrate crystalline form of Moxifloxacin hydrochloride, herein referred to as Form α1. The crystalline Form α1 of Moxifloxacin hydrochloride is characterized by an XRPD pattern comprising peaks at about 5.6, 7.0, 8.3, 9.9, 14.3, 15.4, 17.2, 20.2, 23.4, 26.4, 27.3, 29.0 ± 0.2° 2θ, as depicted in Figure 1; and a DSC endotherm maximum peak at about 250 °C, as depicted in Figure 2.

In another aspect, the invention provides a process for the preparation of the hydrate Form α1 of Moxifloxacin hydrochloride comprising the steps of:
a) suspending Moxifloxacin hydrochloride in aqueous hydrochloric acid;
b) heating until dissolution;
c) cooling to obtain a precipitate;
d) recovering the product.

The starting Moxifloxacin hydrochloride may be either in amorphous form or in any crystalline form. The Moxifloxacin hydrochloride was suspended in aqueous hydrochloric acid. Preferably, the ratio Moxifloxacin hydrochloride/HCl is from 1:1 to 1:1.5 mole/mole. Preferably, the ratio Moxifloxacin hydrochloride/water is from 1:4 to 1:6 weight/volume.

The suspension was heated in order to dissolve completely Moxifloxacin hydrochloride. Preferably, the solution is heated to a temperature comprised in a range of about 90-100 °C.

The solution was then cooled to obtain a precipitate. Preferably, the system is cooled to room temperature, more preferably is cooled to a temperature comprised in a range of about 15-25 °C. The product was then recovered by methods well known to those skilled in the art.

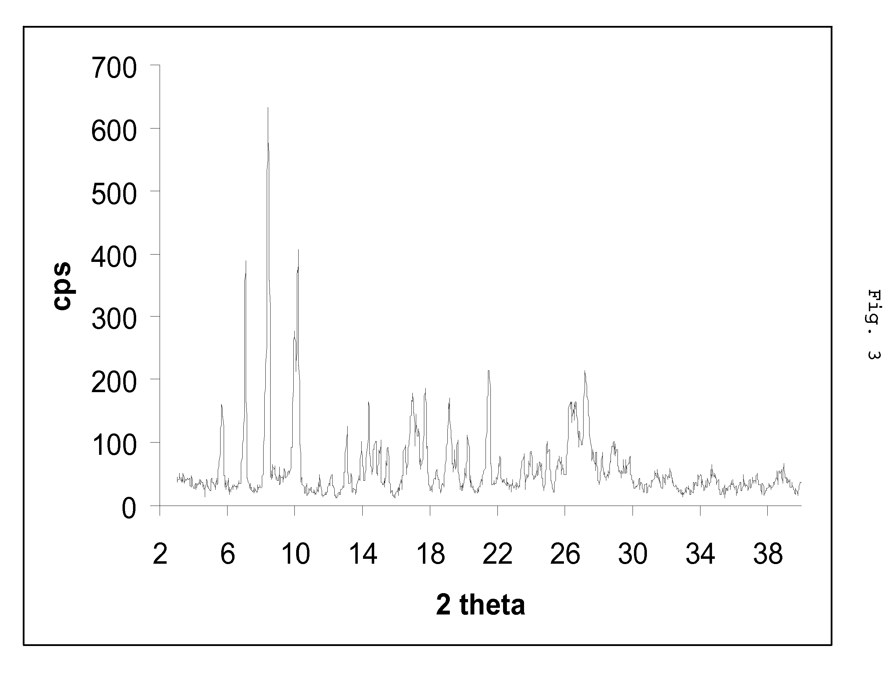

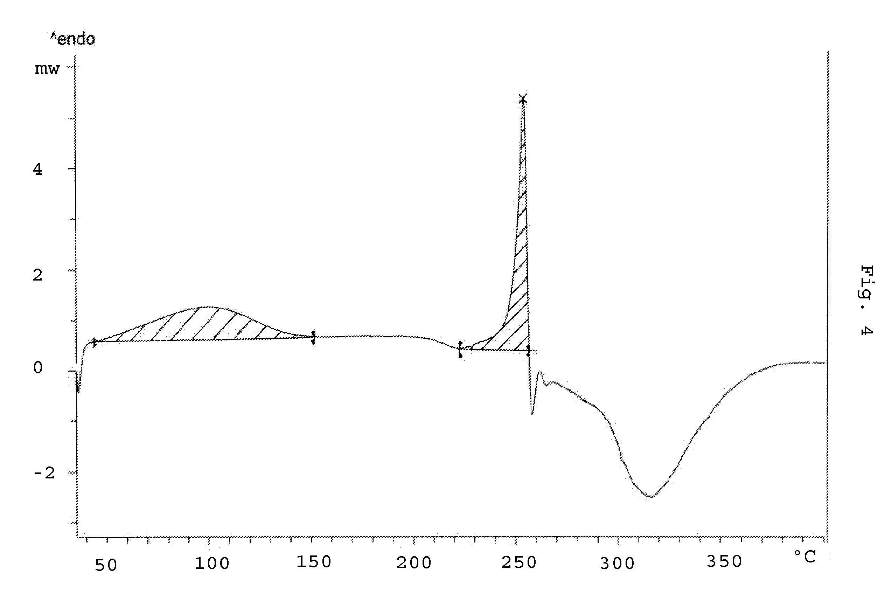

In another aspect, the invention provides a novel hydrate crystalline form of Moxifloxacin hydrochloride, herein referred to as Form α2. The hydrate crystalline Form α2 of Moxifloxacin hydrochloride is characterized by an XRPD pattern comprising peaks at about 5.7, 7.1, 8.4, 10.2, 14.4, 16.9, 19.1, 21.5, 26.3, 27.2 ± 0.2° 2θ, as depicted in Figure 3; and a DSC endotherm maximum peak at about 253 °C, as depicted in Figure 4.

In another aspect, the invention provides a process for the preparation of Form α2 of Moxifloxacin hydrochloride comprising the steps of:
a) suspending Moxifloxacin hydrochloride in a mixture of water and at least an aprotic polar solvent;
b) heating until dissolution;
c) cooling to obtain a precipitate;
d) recovering the product.

The starting Moxifloxacin hydrochloride may be either in amorphous form or in any crystalline form. The Moxifloxacin hydrochloride was suspended in a mixture of water and at least an aprotic polar solvent. Examples of aprotic polar solvents include but are not limited to amides, such as N,N-dimethylacetamide, 2-pyrrolidinone, N-methyl-2-pyrrolidinone or caprolactam and/or mixture thereof. Preferably, suitable solvents are 2-pyrrolidinone or N-methyl-2-pyrrolidinone. More preferably, the solvent is N-methyl-2-pyrrolidinone. Preferably, the mixture of water and aprotic polar solvent has an overall water content comprised in a range of about 0.1% to 2.5% by weight. Preferably, Moxifloxacin hydrochloride is in a ratio of about 1:3 to 1:5 weight/volume with respect to the mixture of water and aprotic polar solvent.

The suspension was heated in order to dissolve completely Moxifloxacin hydrochloride. Preferably, the solution is heated to a temperature comprised in a range of about 120-140 °C.

The solution was then cooled to obtain a precipitate. Preferably, the system is cooled to room temperature, more preferably is cooled to a temperature comprised in a range of about 15-25 °C. The product was then recovered by methods well known to those skilled in the art.

The crystalline forms of the present invention may be recovered by methods well known to those skilled in the art for the separation of the crystallized solid from the mother liquor, for example by filtration, with or without the assistance of pressure and/or vacuum, or by centrifugation, or by decantation. The collected solid is washed with at least a solvent and dried by conventional means.

One skilled in the art will appreciate that by adjusting concentration, temperature and time the yield of the hydrate crystalline Forms α1 and α2 of Moxifloxacin hydrochloride may be optimized.

As mentioned above the compounds of the invention have useful therapeutic properties.

In another aspect, the invention provides a hydrate crystalline Form α1 of Moxifloxacin hydrochloride for use as a medicament.

In another aspect, the invention provides the use of a hydrate crystalline Form α1 of Moxifloxacin hydrochloride for the preparation of a medicament for the treatment of infections.

In another aspect, the invention provides a hydrate crystalline Form α2 of Moxifloxacin hydrochloride for use as a medicament.

In another aspect, the invention provides the use of a hydrate crystalline Form α2 of Moxifloxacin hydrochloride for the preparation of a medicament for the treatment of infections.

Hydrate crystalline Form α1 or Form α2 may be administered per se or, preferably, as a pharmaceutical composition. The polymorphic forms of the present invention may be used as single components or mixtures. Moxifloxacin hydrochloride may be present in the pharmaceutical formulations as the only active compound or can be combined with other active substances.

In another aspect, the invention provides a pharmaceutical composition comprising a hydrate crystalline Form α1 optionally together with at least one pharmaceutically acceptable excipient.

In another embodiment, the invention provides a pharmaceutical composition comprising a hydrate crystalline Form α2 optionally together with at least one pharmaceutically acceptable excipient.

Excipients include, by way of illustration and not limitation, diluents, fillers, agglutinants, disintegrants, disintegration inhibitors, absorption accelerators, binders, carriers, suspensing/dispersing agents, film formers/coatings, adhesives, antiadherents, wetting agents, lubricants, glidants, preservatives, sorbents, surface active agents, substances added to mask or counteract a disagreeable taste or odor, flavorings, colorants, fragrances, aromatising agents, sweeteners and substances added to improve appearance of the composition.

A person skilled in the art is aware of a whole variety of such excipient compounds suitable to formulate a pharmaceutical composition. The choice of excipient will to a large extent depend on factors such as the particular mode of administration, the effect of the excipient on solubility and stability, and the nature of the dosage form.

A hydrate crystalline Form α1 or Form α2 of Moxifloxacin hydrochloride, together with a conventionally employed excipient, may be placed into the form of pharmaceutical compositions and unit dosages thereof, and in such form may be employed as solids, such as tablets, capsules, pills, powders, granules, pellets, lozenges, pastilles, or liquids, such as solutions, suspensions, emulsions, drops, lotions, sprays, tinctures, syrups, elixirs, or capsules filled with the same, all for oral use, or in the form of sterile injectable solutions for parenteral (including subcutaneous, intramuscular, and intravenous) use, or in the form of suppositories for rectal use, or in the form of creams, ointments, gels, unguents for topical use and other forms suitable for the inhalatory or transdermal administrations. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

Pharmaceutical compositions containing a hydrate crystalline Form α1 or Form α2 of Moxifloxacin hydrochloride can be prepared in a manner well known in the pharmaceutical art and comprise at least one active compound. Generally, the compounds of this invention are administered in a pharmaceutically effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

The pharmaceutical compositions of the present invention can be administered by a variety of routes including oral, rectal, parenteral (including subcutaneous, intravenous, intramuscular), topical, transdermal, ophtalmic and intranasal. The compositions for oral administration can take the form of bulk liquid solutions or suspensions, or bulk powders. More commonly, however, the compositions are presented in unit dosage forms to facilitate accurate dosing. Typical unit dosage forms include prefilled, premeasured ampoules or syringes of the liquid compositions or pills, tablets, capsules, powders, granules, pellets, lozenges, pastilles, suppositories or the like in the case of solid compositions. In such compositions, a hydrate crystalline Form α1 or Form α2 of Moxifloxacin hydrochloride is usually a minor component (from about 0.1 to about 50% by weight or preferably from about 1 to about 40% by weight) with the remainder being various vehicles or carriers and processing aids helpful for forming the desired dosing form.

Solid forms may include, for example, any of the following ingredients, or compounds of a similar nature: a binder, such as cellulose-derived materials like powdered cellulose, microcrystalline cellulose, microfine cellulose, methyl cellulose, ethyl cellulose, hydroxyethyl cellulose, hydroxypropyl cellulose, hydroxypropylmethyl cellulose, carboxymethyl cellulose salts and other substituted and unsubstituted celluloses, gum tragacanth or gelatine; an excipient, such as starch or lactose, a disintegrating agent, such as alginic acid, primogel, or corn starch; a lubricant, such as magnesium stearate, hydrogenated castor oil, polyethylene glycol; a glidant, such as colloidal silicon dioxide; a sweetening agent, such as sorbitol, sucrose, aspartame or saccharin; or a flavoring agent, such as maltol, vanillin, menthol, citric acid, pepper-mint, methyl salicylate, or orange flavoring.

Liquid forms suitable for oral administration may include a suitable aqueous or non-aqueous vehicle with buffers, suspending and dispersing agents, colorants, flavors and the like.

Injectable compositions are typically based upon injectable sterile saline or phosphate-buffered saline or other injectable carriers known in the art. As above mentioned, a hydrate crystalline Form α1 or Form α2 of Moxifloxacin hydrochloride in such compositions is typically a minor component, with the remainder being the injectable carrier and the like.

The above described components for orally administered or injectable compositions are merely representative. Further materials as well as processing techniques and the like are set out in Part 5 of Remington 's Pharmaceutical Sciences, 20th Edition, 2000, Merck Publishing Company, Easton, Pennsylvania, which is incorporated herein by reference. A hydrate crystalline Form α1 or Form α2 of Moxifloxacin hydrochloride of this invention can also be administered in sustained release forms or from sustained release drug delivery systems. A description of representative sustained release materials can also be found in the incorporated materials in Remington 's Pharmaceutical Sciences.

While the present invention has been described in terms of its specific embodiments, certain modifications and equivalents will be apparent to those skilled in the art and are included within the scope of the present invention.

In the following, the present invention shall be illustrated by means of some examples, which are not construed to be viewed as limiting the scope of the invention.

### Example 1

### Preparation of Form α1 of Moxifloxacin hydrochloride

Moxifloxacin hydrochloride (10 g) was suspended in water (50 ml) with 37% aqueous hydrochloric acid (3 g). The suspension was heated to 100 °C until complete dissolution was observed. The solution was cooled to 20 °C and the attained solid material was filtered, washed with water and dried under reduced pressure at 95 °C, affording Form α1 of Moxifloxacin hydrochloride (8.9 g), having a water content of 3.64% and a XRPD pattern and a DSC thermogram as substantially depicted in Figure 1 and Figure 2.

### Example 2

### Preparation of Form α2 of Moxifloxacin hydrochloride

Moxifloxacin hydrochloride (10 g) was suspended in N-methyl-2-pyrrolidinone (40 ml) and water (0.5 ml). The suspension was heated to 130 °C, then cooled to 20 °C. The attained solid material was filtered, washed with acetone and dried under reduced pressure at 60 °C, affording Form α2 of Moxifloxacin hydrochloride (7.3 g), having a water content of 4.02 % and a XRPD pattern and a DSC thermogram as substantially depicted in Figure 3 and Figure 4.

## Claims

1. A hydrate crystalline form of Moxifloxacin hydrochloride (Form α1) having an X-ray powder diffraction pattern comprising peaks at about 5.6, 7.0, 8.3, 9.9, 14.3, 15.4, 17.2, 20.2, 23.4, 26.4, 27.3, 29.0 ± 0.2° 2θ.

2. A hydrate crystalline form according to claim 1, substantially **characterized by** an X-ray powder diffraction pattern in accordance with Figure 1.

3. A hydrate crystalline form according to claim 1, **characterized by** a differential scanning calorimetry endothermic maximum peak at about 250 °C.

4. A hydrate crystalline form according to claim 1, substantially **characterized by** a differential scanning calorimetry endothermic in accordance with Figure 2.

5. A hydrate crystalline form according to claim 1, wherein the water content is from 3.5 to 4.2 % w/w.

6. A process for preparing the crystalline form according to claim 1, which comprises the steps of:
a) suspending Moxifloxacin hydrochloride in aqueous hydrochloric acid;
b) heating until dissolution;
c) cooling to obtain a precipitate;
d) recovering the product.

7. A process according to claim 6, step a), wherein the ratio Moxifloxacin hydrochloride/HCl is from 1:1 to 1:1.5 mole/mole.

8. A process according to claim 6, step a), wherein the ratio Moxifloxacin hydrochloride/water is from 1:4 to 1:6 weight/volume.

9. A hydrate crystalline form according to claim 1, for use as a medicament.

10. Use of a hydrate crystalline form according to claim 1, for the preparation of a medicament for the treatment of infections.

11. A pharmaceutical composition comprising the hydrate crystalline form according to claim 1, optionally together with at least one pharmaceutically acceptable excipient.

12. A hydrate crystalline form of Moxifloxacin hydrochloride (Form α2) having an X-ray powder diffraction pattern comprising peaks at about 5.7, 7.1, 8.4, 10.2, 14.4, 16.9, 19.1, 21.5, 26.3, 27.2 ± 0.2° 2θ.

13. A hydrate crystalline form according to claim 12, substantially **characterized by** an X-ray powder diffraction pattern in accordance with Figure 3.

14. A hydrate crystalline form according to claim 12, **characterized by** a differential scanning calorimetry endothermic maximum peak at about 253 °C.

15. A hydrate crystalline form according to claim 12, substantially **characterized by** a differential scanning calorimetry endothermic in accordance with Figure 4.

16. A hydrate crystalline form according to claim 12, wherein the water content is from 3.5 to 4.2 % w/w.

17. A process for preparing the crystalline form according to claim 12, which comprises the steps of:
a) suspending Moxifloxacin hydrochloride in a mixture of water and at least an aprotic polar solvent;
b) heating until dissolution;
c) cooling to obtain a precipitate;
d) recovering the product.

18. A process according to claim 17, step a), wherein the aprotic polar solvent is an amide.

19. A process according to claim 18, wherein the amide is N,N-dimethylacetamide, 2-pyrrolidinone, N-methyl-2-pyrrolidinone or caprolactam and/or mixture thereof.

20. A process according to claim 19, wherein the amide is 2-pyrrolidinone or N-methyl-2-pyrrolidinone.

21. A process according to claim 20, wherein the amide is N-methyl-2-pyrrolidinone.

22. A process according to claim 17, wherein the mixture of water and aprotic polar solvent has an overall water content comprised in a range of about 0.1-2.5% by weight.

23. A process according to claim 17, wherein Moxifloxacin hydrochloride is in a ratio of about 1:3 to 1:5 weight/volume with respect to the mixture of water and aprotic polar solvent.

24. A hydrate crystalline form according to claim 12, for use as a medicament.

25. Use of a hydrate crystalline form according to claim 12, for the preparation of a medicament for the treatment of infections.

26. A pharmaceutical composition comprising the hydrate crystalline form according to claim 12, optionally together with at least one pharmaceutically acceptable excipient.
